# EUROPEAN PATENT APPLICATION

(11) **EP 4 538 267 A1**
(43) Date of publication of application: **16.04.2025**
(21) Application number: 23823653.3
(22) Date of filing: 25.05.2023
(51) Int. Cl.: C07D 403/04, C07D 403/14, C09B 57/04, C09B 67/20

(54) **ISOINDOLINE COMPOUND**

(30) Priority: 13.06.2022 JP 2022095018
(71) Applicant: DIC Corporation, Tokyo 174-8520 (JP)
(72) Inventor: FOO Siongwan, Tokyo 174-8520 (JP); EBATO Hiroshi, Tokyo 174-8520 (JP)
(74) Representative: TBK
(86) International application number: PCT/JP2023/019515
(87) International publication number: WO 2023/243354

(57) **Abstract**

Provided are a bio-derived isoindoline compound that can be used as an isoindoline-based yellow pigment and obtained by a safe, clean and green, and carbon-neutral approach, and a composition containing the isoindoline compound. The isoindoline compound contains a radiocarbon atom ¹⁴C and has a group represented by the following formula (A) .

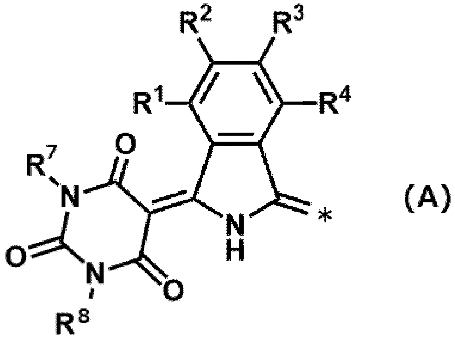

(In the formula (A), R¹ to R⁴ each independently represent a hydrogen atom, a linear or branched alkyl group having 1 to 12 carbon atoms, a fluorine atom, a chlorine atom, a bromine atom, an iodine atom, or a phenyl group that is optionally substituted with an alkyl group. Note that R² and R³ are optionally ring-closed to form a 5- to 8-membered ring. R⁷ and R⁸ each independently represent a hydrogen atom, a linear or branched alkyl group having 1 to 12 carbon atoms, or a phenyl group that is optionally substituted with an alkyl group. * represents a bonding hand.)

## Description

### Technical Field

The present invention relates to an isoindoline compound, a composition containing the isoindoline compound, and a method for producing the isoindoline compound.

### Background Art

As for an isoindoline-based yellow pigment, the molecular thereof is not as large as that of an azo dimer and it has relatively high fastness to light and heat even though halogens are not included. Therefore, it has high potential in many applications, including automotive coatings, decorative paints, GI coatings, plastics, inks, specialty applications, and the like.

Commercially available isoindoline-based yellow pigments are prepared by coupling 1,3-diiminoisoindoline (DII) with a specific building block. DII is mainly synthesized using phthalonitrile as a starting raw material (for example, see NPL 1), and, in addition, it has been also reported that phthalic anhydride is used (for example, see PTL 1) as a starting raw material.

However, phthalonitrile is toxic to the human body and also has adverse effects on the environment. Also, generally, the only source of supply of phthalonitrile is petroleum resources.

In recent years, there has been a growing trend toward switching from petroleum-derived raw materials to bio (biomass)-derived raw materials and replacing harmful chemicals with less toxic ones from the viewpoint of sustainable development, a reduction of carbon dioxide emission amount, and safety.

Currently produced isoindoline-based yellow pigments are not likely to meet the requirements of future environmental issues, and it is desirable to provide bio-derived isoindoline-based yellow pigments from the viewpoint of being clean and green, and being carbon-neutral.

### Citation List

### Patent Literature

PTL 1: Japanese Unexamined Patent Application Publication No. 7-330729

### Non Patent Literature

NPL 1: Chem. Lett., 1984, 8, 1423-1426

### Summary of Invention

### Technical Problem

An object of the present invention is to provide a bio-derived isoindoline compound obtained by a safe, clean and green, and carbon-neutral approach that can be used as an isoindoline-based yellow pigment, and a composition containing the isoindoline compound.

### Solution to Problem

The present inventor has investigated to solve the above problem, and as a result, found that bio-derived furan and bio-derived maleic anhydride are used as a raw material to obtain a DA intermediate by the Diels-Alder (DA) reaction, and the DA intermediate is dehydroaromatized and aminated with urea to obtain bio-DII containing a radiocarbon atom ¹⁴C, and the bio-DII is then coupled with building blocks, resulting in obtaining a bio-derived isoindoline compound that can be used as an isoindoline-based yellow pigment. Thus, the present invention has been completed.

In other words, the present invention encompasses the following aspects.
[1] An isoindoline compound containing a radiocarbon atom ¹⁴C and having a group represented by the following formula (A). 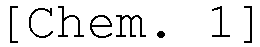 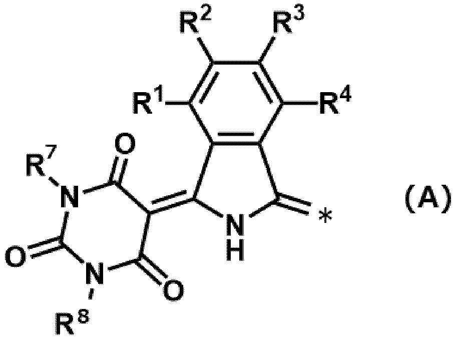 (In the formula (A), R¹ to R⁴ each independently represent a hydrogen atom, a linear or branched alkyl group having 1 to 12 carbon atoms, a fluorine atom, a chlorine atom, a bromine atom, an iodine atom, or a phenyl group that is optionally substituted with an alkyl group. Note that R² and R³ are optionally ring-closed to form a 5- to 8-membered ring. R⁷ and R⁸ each independently represent a hydrogen atom, a linear or branched alkyl group having 1 to 12 carbon atoms, or a phenyl group that is optionally substituted with an alkyl group. * represents a bonding hand.)
[2] The isoindoline compound according to item [1], in which the isoindoline compound having a group represented by the formula (A) is an isoindoline compound represented by the following formula (A-1) or the following formula (A-2). 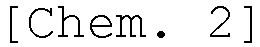 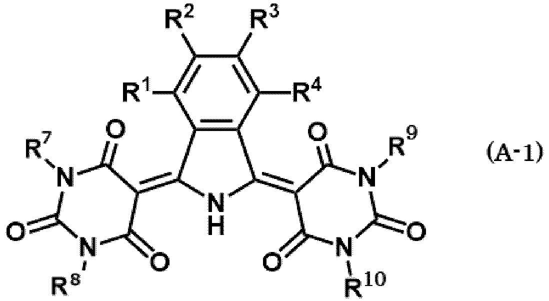 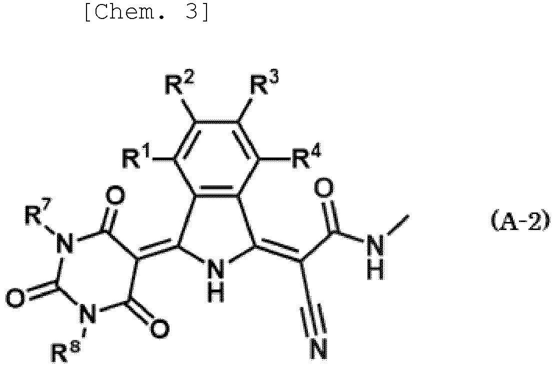 (In the formula (A-1) and the formula (A-2), R¹ to R⁴ represent the same as R¹ to R⁴ above. R⁷ and R⁸ represent the same as R⁷ and R⁸ above. R⁹ and R¹⁰, similar to R⁷ and R⁸ above, each independently represent a hydrogen atom, a linear or branched alkyl group having 1 to 12 carbon atoms, or a phenyl group that is optionally substituted with an alkyl group.)
[3] The isoindoline compound according to item [2], in which the isoindoline compound represented by the formula (A-1) is an isoindoline compound represented by the following formula (A-1-1), or the isoindoline compound represented by the formula (A-2) is an isoindoline compound represented by the following formula (A-2-1). 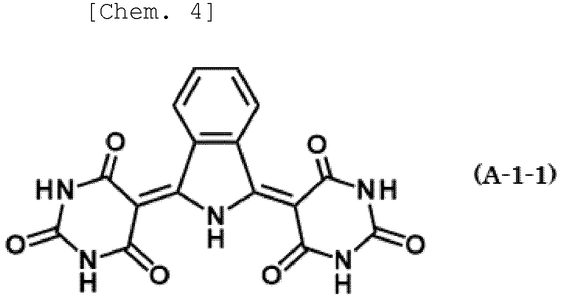 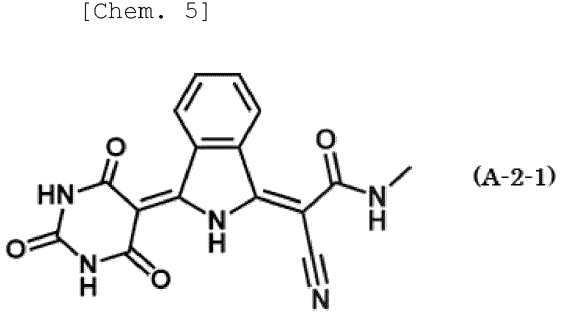
[4] A composition containing the isoindoline compound having a group represented by the formula (A) according to item [1].
[5] The composition according to item [4], used as a composition for pigments.
[6] The composition according to item [4], containing at least two kinds of the isoindoline compounds having a group represented by the formula (A).
[7] The composition according to item [4], containing an isoindoline compound represented by the following formula (A-1) and an isoindoline compound represented by the following formula (A-3). 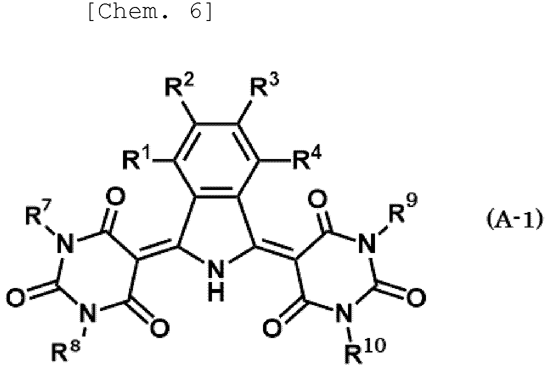 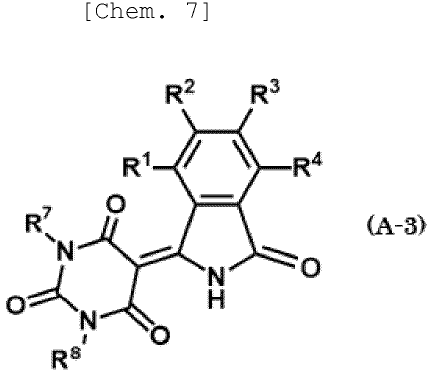 (In the formula (A-1) and the formula (A-3), R¹ to R⁴ each independently represent a hydrogen atom, a linear or branched alkyl group having 1 to 12 carbon atoms, a fluorine atom, a chlorine atom, a bromine atom, an iodine atom, or a phenyl group that is optionally substituted with an alkyl group. Note that R² and R³ are optionally ring-closed to form a 5- to 8-membered ring. R⁷ to R¹⁰ each independently represent a hydrogen atom, a linear or branched alkyl group having 1 to 12 carbon atoms, or a phenyl group that is optionally substituted with an alkyl group.)
[8] The composition according to item [7], in which a ratio of the isoindoline compound represented by the formula (A-3) in the composition is 0.5 to 10.0% by mass.
[9] The composition according to item [7], in which the isoindoline compound represented by the formula (A-1) is an isoindoline compound represented by the following formula (A-1-1), and the isoindoline compound represented by the formula (A-3) is an isoindoline compound represented by the following formula (A-3-1). 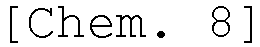 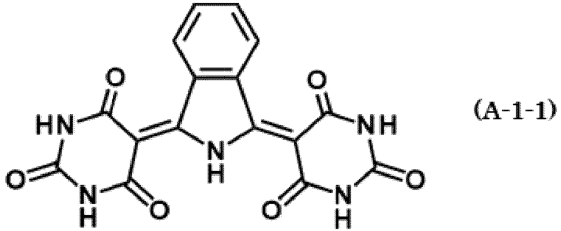 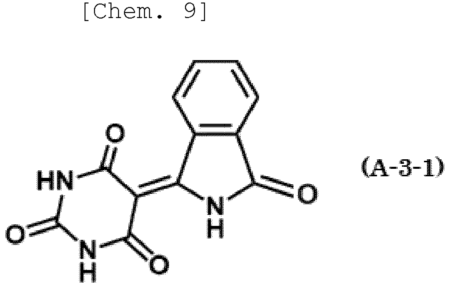
[10] A method for producing an isoindoline compound containing a radiocarbon atom ¹⁴C and having a group represented by the following formula (A), 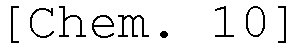 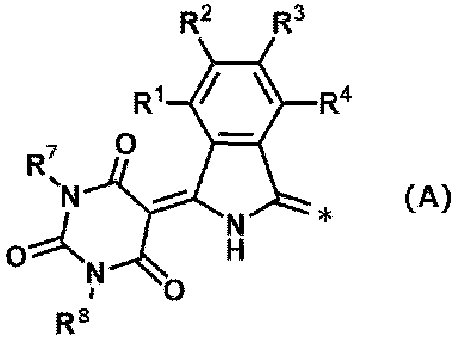
   (In the formula (A), R¹ to R⁴ each independently represent a hydrogen atom, a linear or branched alkyl group having 1 to 12 carbon atoms, a fluorine atom, a chlorine atom, a bromine atom, an iodine atom, or a phenyl group that is optionally substituted with an alkyl group. Note that R² and R³ are optionally ring-closed to form a 5- to 8-membered ring. R⁷ and R⁸ each independently represent a hydrogen atom, a linear or branched alkyl group having 1 to 12 carbon atoms, or a phenyl group that is optionally substituted with an alkyl group. * represents a bonding hand.),
   the method including reacting a compound represented by the following formula (I-2) with barbituric acid represented by the following formula (B) under an acid-free solvent or under a solvent containing at least one kind of acid selected from the group consisting of hydrochloric acid, sulfuric acid, methanesulfonic acid, p-toluenesulfonic acid, acetic acid, triflic acid, phosphoric acid, polyphosphoric acid, pyrophosphoric acid, and nitric acid to obtain an isoindoline compound having a group represented by the formula (A). 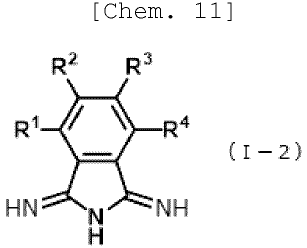
   (In the formula (I), R¹ to R⁴ represent the same as R¹ to R⁴ above.) 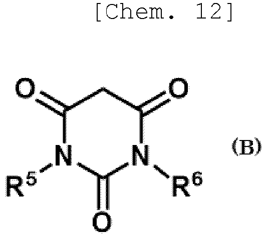
   (In the formula (B), R⁵ and R⁶ each independently represent a hydrogen atom, a linear or branched alkyl group having 1 to 12 carbon atoms, or a phenyl group that is optionally substituted with an alkyl group.)
[11] The method for producing an isoindoline compound according to item [10],
   in which the compound represented by the formula (I-2) is obtained by obtaining a compound represented by the following formula (PA) from a compound represented by the following formula (DA), 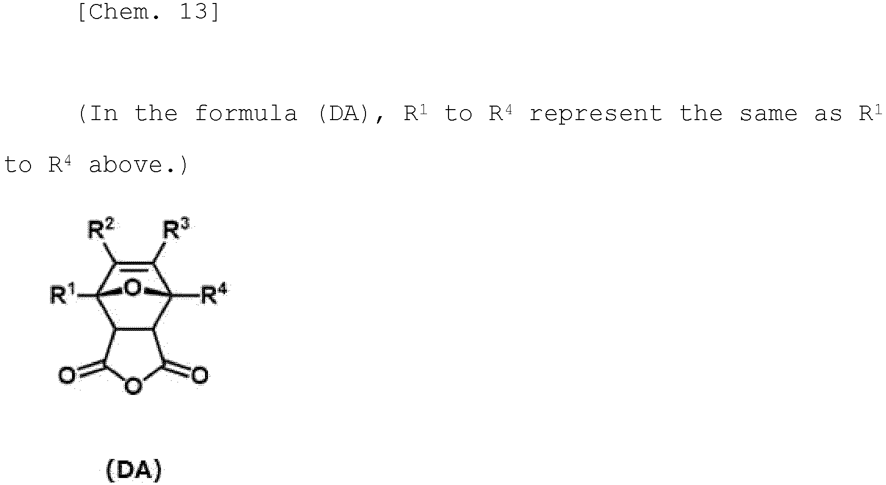 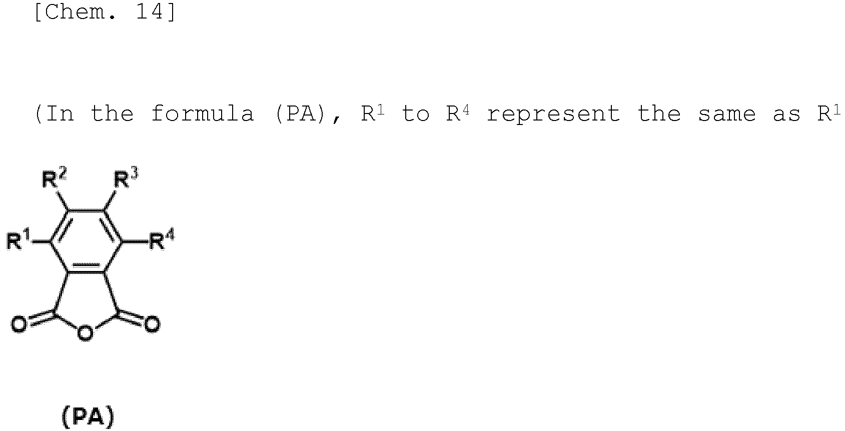
   to R⁴ above.), and
   obtaining an isoindoline compound represented by the formula (I-2) from the compound represented by the formula (PA).
[12] The method for producing an isoindoline compound according to item [10], in which the solvent is any one selected from the group consisting of H₂O, dimethylacetamide (DMA), dimethylformamide (DMF), dimethyl sulfoxide (DMSO), MeOH, EtOH, isopropyl alcohol (IPA), and tBuOH. Advantageous Effects of Invention

The present invention can provide a bio-derived isoindoline compound that can be used as an isoindoline-based yellow pigment and obtained by a safe, clean and green, and carbon-neutral approach, and a composition containing the isoindoline compound.

### Brief Description of Drawings

FIG. 1 is a diagram illustrating the TEM observation result of a pigment in Example 1.
FIG. 2 is a diagram illustrating the TEM observation result of a pigment in Comparative Example 1.

### Description of Embodiments

Hereinafter, the present invention will be described in details. Note that the description of the constituent requirements described below is an example to explain the present invention, and the present invention is not limited to these contents.

### (Isoindoline Compound Containing Radiocarbon Atom ¹⁴C and Having Group Represented by Following Formula (A))

The isoindoline compound of the present invention contains a radiocarbon atom ¹⁴C.

The isoindoline compound of the present invention have a group represented by the following formula (A).

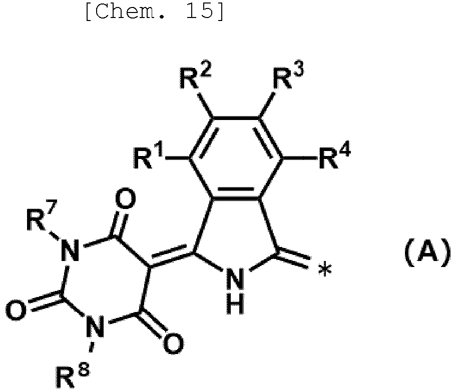

In the formula (A), R¹ to R⁴ each independently represent a hydrogen atom, a linear or branched alkyl group having 1 to 12 carbon atoms, a fluorine atom, a chlorine atom, a bromine atom, an iodine atom, or a phenyl group that is optionally substituted with an alkyl group. Note that R² and R³ are optionally ring-closed to form a 5- to 8-membered ring. R⁷ and R⁸ each independently represent a hydrogen atom, a linear or branched alkyl group having 1 to 12 carbon atoms, or a phenyl group that is optionally substituted with an alkyl group. * represents a bonding hand.

The isoindoline compound of the present invention is produced using a bio-isoindoline compound containing a radiocarbon atom ¹⁴C represented by the following formula (I-2), which is produced using bio-derived furan and bio-derived maleic anhydride, and therefore contains bio (biomass)-components derived from bio raw materials. The presence of bio-components in the isoindoline compounds of the present invention can be checked, for example, by measuring the radiocarbon atom ¹⁴C.

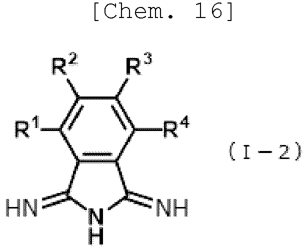

In the formula (I-2), R¹ to R⁴ represent the same as R¹ to R⁴ above.

For example, organism-derived and petroleum-derived compounds and compositions do not differ in physical properties such as a molecular weight, and mechanical and thermal properties. To distinguish between them, the biomass degree is generally used. As to this biomass degree, ¹⁴C (radiocarbon 14, half-life 5,730 years) is not contained in the carbon in the petroleum-derived compounds and compositions, and therefore the concentration of this ¹⁴C can be measured by an accelerator mass spectrometry to check whether the compounds and compositions produced are a petroleum-derived compound or a bio-derived compound.

In this biomass degree measurement, for example, a measuring objective sample is burned to generate carbon dioxide, the generated carbon dioxide is purified in a vacuum line, and then reduced with hydrogen using iron as a catalyst to produce graphite.

The graphite is then mounted on a ¹⁴C-AMS dedicated device (manufactured by NEC Corporation) based on a tandem accelerator to measure ¹⁴C counts, a ¹³C concentration (¹³C/¹²C), and a ¹⁴C concentration (¹⁴C/¹²C), and from the obtained measured values, the ratio of ¹⁴C concentration of the sample carbon to the standard modern carbon is calculated to obtain the biomass degree. Note that, in the measurement, oxalic acid (HOxII) provided by the National Institute of Standards and Technology (NIST) can be used as a standard sample.

One indicator to evaluate the biomass degree is, for example, the percent Modern Carbon (pMC).

Here, the percent Modern Carbon (pMC) can be calculated by measuring according to ASTM-D6866-18 and represents a ratio of the ¹⁴C concentration in an object to the ¹⁴C concentration in standard modern carbon. Note that, in a case where the object does not contain radiocarbon atom ¹⁴C, the pMC is 0%.

In the formula (I), R² and R³ are optionally ring-closed to form a 5- to 8-membered ring, as described above. Then, the ring structure can be a "functional" etheric, alcoholic, or carboxylic functional group. Here, the term a "functional" functional group means that a -OH group, a - COOH group, and a -O- group may be included in the ring structure. For example, it means that the compounds may be represented by the following formulae (v), (vi), and (vii).

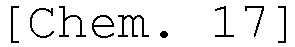

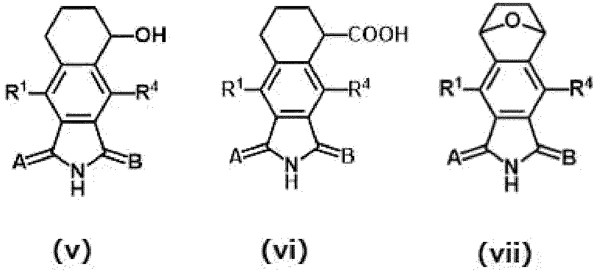

Preferred embodiments of the isoindoline compound having a group represented by the formula (A) include, for example, an isoindoline compound represented by the following formula (A-1) and an isoindoline compound represented by the following formula (A-2).

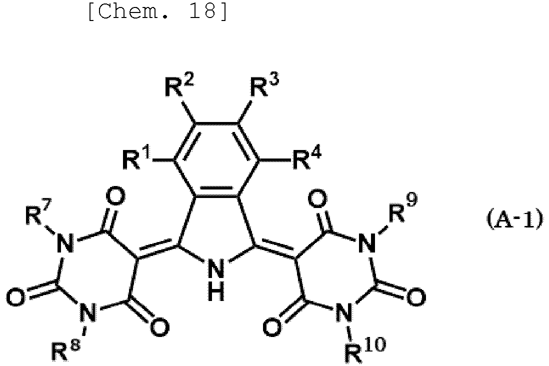

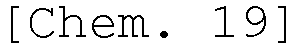

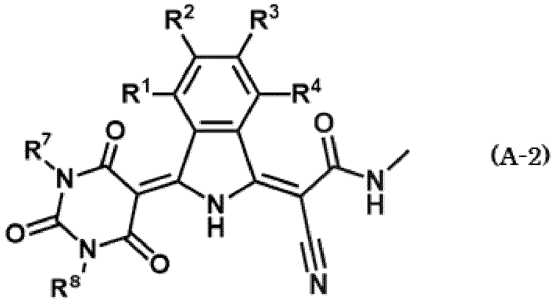

In the formula (A-1) and the formula (A-2), R¹ to R⁴ represent the same as R¹ to R⁴ above. R⁷ and R⁸ represent the same as R⁷ and R⁸ above. R⁹ and R¹⁰, similar to R⁷ and R⁸ above, each independently represent a hydrogen atom, a linear or branched alkyl group having 1 to 12 carbon atoms, or a phenyl group that is optionally substituted with an alkyl group.

The percent Modern Carbon (pMC) in the isoindoline compound represented by the formula (A-1) and the isoindoline compound represented by the formula (A-2) is preferably 1% or more, more preferably 50% or more, more preferably 75% or more, more preferably 90% or more, and even more preferably 99% or more.

Furthermore, preferred embodiments of the isoindoline compounds represented by the formula (A-1) include, for example, an isoindoline compound represented by the following formula (A-1-1), and preferred embodiments of the isoindoline compounds represented by the formula (A-2) include, for example, an isoindoline compound represented by the following formula (A-2-1). Note that the isoindoline compound represented by the formula (A-1-1) is a compound known to constitute a Y139 pigment, and the isoindoline compound represented by the formula (A-2-1) is a compound known to constitute a Y185 pigment.

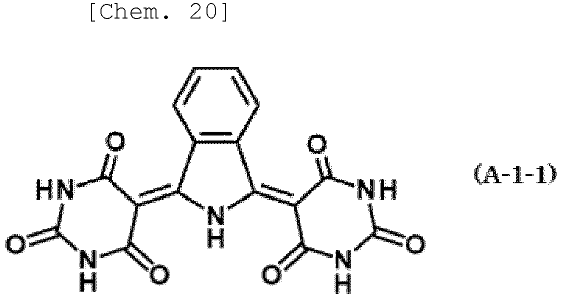

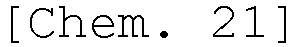

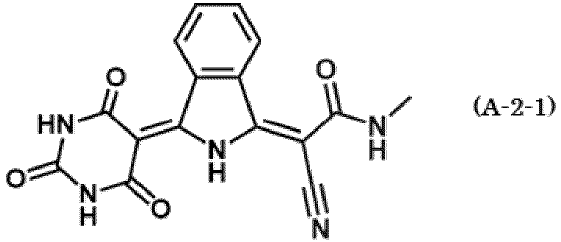

The isoindoline compound of the present invention containing a group represented by the formula (A), or a composition containing the isoindoline compound, is used as an isoindoline-based yellow pigment containing a radiocarbon atom ¹⁴C, that is, a bio-component.

### (Method For Producing Isoindoline Compound Containing Group Represented By Formula (A))

The isoindoline compound of the present invention containing a radiocarbon atom ¹⁴C, that is, containing a bio-component and containing a group represented by the formula (A), can be obtained by the method described below.

The isoindoline compound having a group represented by the formula (A) can be obtained by reacting the compound represented by the following formula (I-2) with barbituric acid represented by the following formula (B), more preferably under a solvent containing an acid, although the reaction proceeds without an acid.

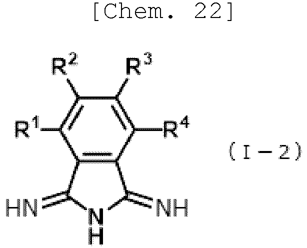

In the formula (I-2), R¹ to R⁴ represent the same as R¹ to R⁴ above.

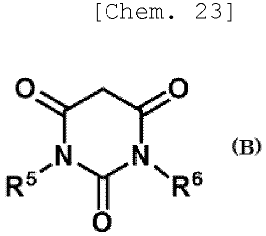

In the formula (B), R⁵ and R⁶ each independently represent a hydrogen atom, a linear or branched alkyl group having 1 to 12 carbon atoms, or a phenyl group that is optionally substituted with an alkyl group.

Preferred embodiments of the method for producing the isoindoline compound having a group represented by the formula (A) include a production method in which the isoindoline compound is particularly an isoindoline compound represented by the formula (A-1).

The isoindoline compound represented by the formula (A-1) can be obtained by the method described below.

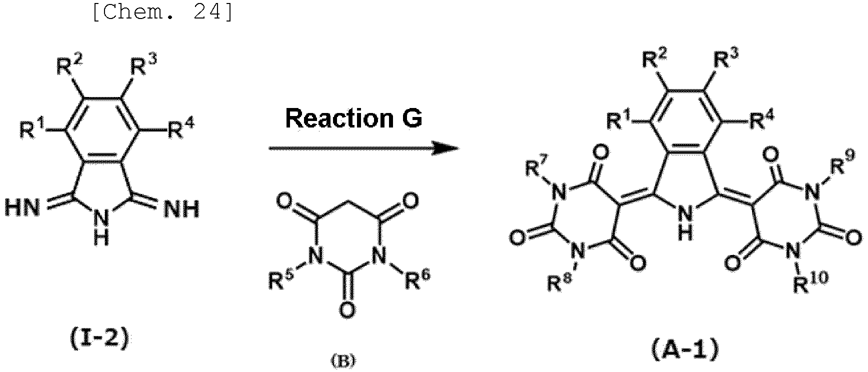

In the formula (I-2) and the formula (A-1), R¹ to R¹⁰ represent the same as R¹ to R¹⁰ above.

### <Reaction G>

The isoindoline compound represented by the formula (A-1) can be obtained by reacting the compound represented by the formula (I-2) with barbituric acid represented by the formula (B), more preferably under a solvent containing an acid (more specifically, under a solvent containing at least one kind of acid selected from the group consisting of hydrochloric acid, sulfuric acid, methanesulfonic acid, p-toluenesulfonic acid, acetic acid, triflic acid, phosphoric acid, polyphosphoric acid, pyrophosphoric acid, and nitric acid), or under a solvent without containing an acid, although the reaction proceeds without an acid.

The concentration of acid used in the reaction G is preferably from 0.5 to 30% by mass, and more preferably from 1 to 10% by mass.

The solvent used when reacting barbituric acid represented by the formula (B) is preferably any one selected from the group consisting of H₂O, dimethylacetamide (DMA), dimethylformamide (DMF), dimethylsulfoxide (DMSO), MeOH, EtOH, isopropyl alcohol (IPA), and tBuOH.

In the isoindoline compound having a group represented by the formula (A-1) obtained by the reaction G, R⁷ and R⁸ correspond to R⁵ and R⁶ in the formula (B), respectively, but R⁷ may be R⁵ and R⁸ may be R⁶, or R⁷ may be R⁶ and R⁸ may be R⁵. Also, similarly, R⁹ and R¹⁰ correspond to R⁵ and R⁶ in the formula (B), respectively, but R⁹ may be R⁵ and R¹⁰ may be R⁶, or R⁹ may be R⁶ and R¹⁰ may be R⁵.

Note that the compound represented by the formula (I-2) is obtained by obtaining a compound represented by the following formula (PA) from a compound represented by the following formula (DA),

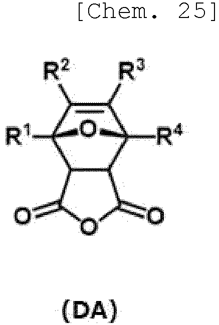

(In the formula (DA), R¹ to R⁴ represent the same as R¹ to R⁴ above.)

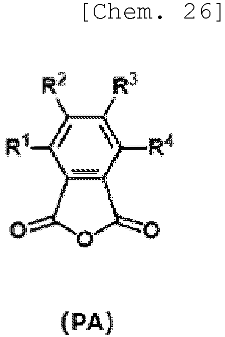

(In the formula (PA), R¹ to R⁴ represent the same as R¹ to R⁴ above.), and
obtaining an isoindoline compound represented by the formula (I-2) from the compound represented by the formula (PA).

When obtaining the compound represented by the formula (PA) from the compound represented by the formula (DA), the reaction can be carried out under an acidic condition.

Any acidic condition is acceptable as long as it allows the reaction to proceed suitably, but, for example, the compound represented by the formula (PA) is formed using any acid selected from the group consisting of hydrochloric acid, sulfuric acid, methanesulfonic acid, p-toluenesulfonic acid, acetic acid, acetic anhydride, triflic acid, phosphoric acid, phosphoric anhydride, polyphosphoric acid, pyrophosphoric acid, nitric acid, or a mixture of these acids.

When obtaining the isoindoline compound represented by the formula (I-2) from the compound represented by the formula (PA), ammonium molybdenum is used as a catalyst and urea or NH₃ is used as an amine source, and the amination reaction is carried out in the presence of these.

Preferred embodiments of a method for producing an isoindoline compound represented by the formula (I-2) include, for example, a production method described below.

### <Method For Producing Isoindoline Compound Represented By (I-2)>

From a compound represented by the following formula (DA), an isoindoline compound represented by the formula (I-2) can be obtained through a compound represented by the following formula (PA) or the following formula (I-2 Nitrate).

The specific reaction route of this production method is shown below.

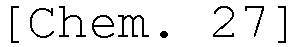

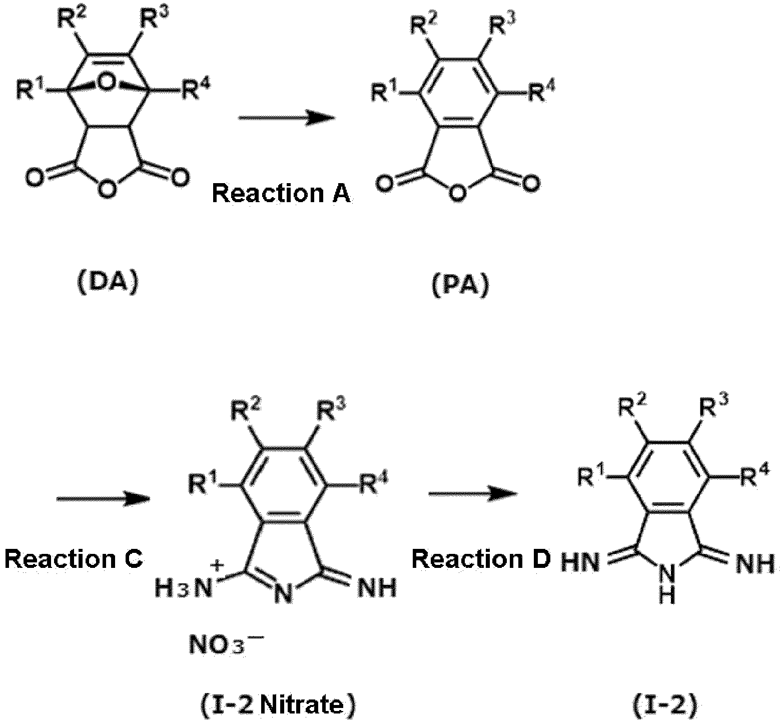

(In the formula, R¹ to R⁴ represent the same as R¹ to R⁴ above.)

### <<Reaction A>>

A compound (DA) is subjected to a ring-opening dehydration reaction thereby being able to produce a compound (PA).

Any reaction solvent is acceptable as long as it allows the reaction to proceed suitably, but the reaction solvent is preferably water, acetonitrile, toluene, xylene, alkylbenzene, or a mixed solvent of these, or no solvent.

Any reaction temperature is acceptable as long as it allows the reaction to proceed suitably, but the reaction temperature is preferably from 20 to 150°C, more preferably from 30 to 120°C, and more preferably from 40 to 100°C. The lower limit value thereof is preferably 20°C or higher, preferably 25°C or higher, preferably 30°C or higher, preferably 35°C or higher, and preferably 40°C or higher. The upper limit value thereof is preferably 150°C or lower, preferably 140°C or lower, preferably 130°C or lower, 120°C or lower, preferably 110°C or lower, and preferably 100°C or lower.

The reaction can be carried out under an acidic condition. Any acidic condition is acceptable as long as it allows the reaction to proceed suitably, but the compound (PA) is formed using any acid selected from the group consisting of hydrochloric acid, sulfuric acid, methanesulfonic acid, p-toluenesulfonic acid, acetic acid, acetic anhydride, triflic acid, phosphoric acid, phosphoric anhydride, polyphosphoric acid, pyrophosphoric acid, nitric acid, or a mixture of these acids.

Among them, acids containing sulfur or phosphorus are preferably used. This is because these acids can be in the δ⁺ and δ⁻ configuration, as represented by the following formula (p).

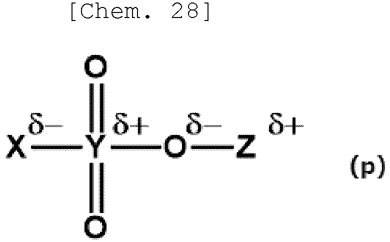

(In the formula (p), X represents -OH, -ONa, -OK, or a methyl group. Y represents a sulfur atom or a phosphorus atom, and Z represents a hydrogen atom, -COCH₃, -COH, or - (PO₂)ₙOH.)

The amount of the acid is preferably 0.1 to 3,000 mol%, preferably 0.5 to 2,500 mol%, preferably 1 to 2,000 mol%, preferably 5 to 1,500 mol%, preferably 10 to 1,000 mol%, preferably 20 to 500 mol%, preferably 50 to 500 mol%, preferably 70 to 500 mol%, preferably 100 to 500 mol%, preferably 150 to 500 mol%, preferably 200 to 500 mol%, preferably 250 to 500 mol%, and preferably 300 to 500 mol%, relative to the compound represented by the formula (DA). The lower limit value thereof is preferably 0.1 mol% or more, preferably 0.5 mol% or more, preferably 1 mol% or more, preferably 5 mol% or more, preferably 10 mol% or more, preferably 20 mol% or more, preferably 50 mol% or more, preferably 70 mol% or more, preferably 100 mol% or more, preferably 150 mol% or more, preferably 200 mol% or more, preferably 250 mol% or more, and preferably 300 mol% or more. The upper limit value thereof is preferably 3,000 mol% or less, preferably 2,500 mol% or less, preferably 2,000 mol% or less, preferably 1,500 mol% or less, preferably 1,000 mol% or less, and preferably 500 mol% or less. Any combination of these upper and lower limit values may be used.

### <<Reaction C>>

After the reaction under an acidic condition as shown in the reaction A, amination can be carried out in a reaction C in the presence of ammonium molybdenum as a catalyst and urea or NH₃ as an amine source.

Note that, in the reaction C, in a case where oxygen atoms in the compound shown in the compound (PA) are substituted with nitrogen atoms, there is no particular need to add any additive if one or two oxygen atoms are substituted, but, in order to replace all oxygen atoms with nitrogen atoms as in the reaction C, a compound represented by the following formula (q) is preferably added to prevent side reactions such as oligomerization and polymerization.

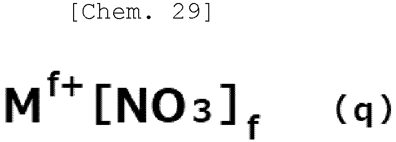

(In the formula (q), M^{f+} represents NH₄⁺, Li⁺, Na⁺, K⁺, Mg²⁺, Ca²⁺, or Al³⁺.)

### <<Reaction D>>

In the reaction D, the isoindoline compound represented by the formula (I-2) can be prepared by providing ammonia water and sodium hydroxide to the reactant obtained through the reaction C.

A composition containing the compound represented by the formula (I-2) can be obtained when producing by the production method, and it is also possible to extract only the compound represented by the formula (I-2) by purification by a known and conventional method.

The compound represented by the formula (DA) used to produce the isoindoline compound represented by the formula (I-2) can be obtained by reacting a compound represented by the following formula (FR) with maleic anhydride.

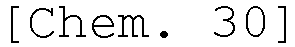

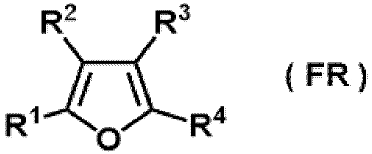

(In the formula (FR), R¹ to R⁴ represent the same as R¹ to R⁴ above.)

The maleic anhydride is preferably derived from biomass.

The compound represented by the formula (FR) is preferably derived from biomass.

Both the maleic anhydride and the compound represented by the formula (FR) are more preferably derived from biomass.

The biomass degree of the raw materials used in the production method is preferably 1% or more, more preferably 50% or more, more preferably 75% or more, more preferably 90% or more, and even more preferably 99% or more. Furthermore, it is easier to achieve the biomass degree of 1% or more and 100% or less in a case where biomass raw materials are available by the mass balance system or the book and claim system. This patent covers the biomass degree including the biomass raw material by the mass balance system or the book and claim system.

Biomass-derived maleic anhydride can be obtained by subjecting maleic acid obtained by oxidizing FF or HMF obtained, for example, by the methods described in Patent No. 5791838 and Patent No. 6328990 to cyclodehydration, or can also be obtained by subjecting maleic acid to direct oxidation. A biomass-derived compound represented by the formula (FR) can be obtained by subjecting FF and HMF obtained, for example, by the methods described in Patent No. 5791838 and Patent No. 6328990 described above to an appropriate combination of a decarbonylation reaction, a reduction reaction, a dehydration reaction, and the like.

The specific reaction route of the production method is shown below.

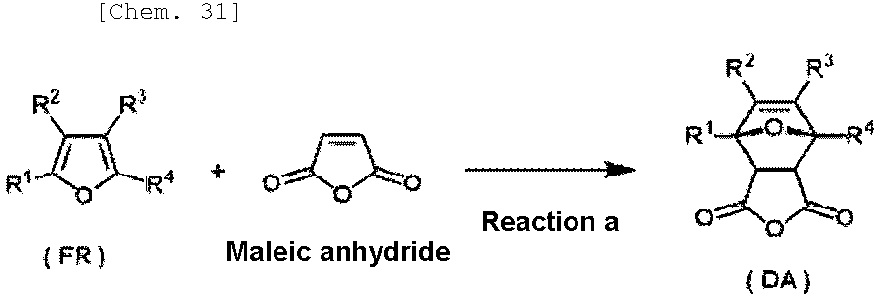

### <<Reaction a>>

The compound (DA) can be produced by subjecting the compound (FR) and maleic anhydride to the Diels-Alder reaction.

The reaction may be carried out using a reaction solvent or using no solvent. Any reaction solvent is acceptable as long as it allows the reaction to proceed suitably, but the reaction solvent is preferably chloroform, dioxane, ethyl acetate, alkylbenzene, toluene, xylene, or diethyl ether.

Any reaction temperature is acceptable as long as it allows the reaction to proceed suitably, but the reaction temperature is preferably from -10 to 100°C, more preferably from 0°C to 80°C, even more preferably from 10°C to 70°C, and particularly preferably from 15°C to 50°C. The lower limit value thereof is preferably -10°C or higher, more preferably 0°C or higher, even more preferably 10°C or higher, and especially preferably 15°C or higher. The upper limit value thereof is preferably 100°C or lower, more preferably 80°C or lower, even more preferably 70°C or lower, and especially preferably 50°C or lower.

Any reaction pressure is acceptable as long as it allows the reaction to proceed suitably, but the reaction pressure is preferably from 0.1 to 5 MPa, more preferably from 0.1 to 3 MPa, even more preferably from 0.1 to 1 MPa, and especially preferably from 0.1 to 0.5 MPa. The lower limit value thereof is preferably 0.1 MPa or more, preferably 0.2 MPa or more, preferably 0.3 MPa or more, and preferably 0.4 MPa or more. The upper limit value thereof is preferably 5 MPa or less, preferably 3 MPa or less, preferably 1 MPa or less, preferably 0.9 MPa or less, preferably 0.8 MPa or less, preferably 0.7 MPa or less, preferably 0.6 MPa or less, and preferably 0.5 MPa or less.

### (Composition Containing Isoindoline Compound)

The present invention may be a composition containing an isoindoline compound of the present invention having a group represented by the formula (A).

The composition is preferably used as a composition for pigments, especially as an isoindoline-based yellow pigment.

The composition of the present invention can, for example, contain one kind or two or more kinds of isoindoline compounds having a group represented by the formula (A).

More preferred embodiments of the composition of the present invention include, for example, a composition containing an isoindoline compound represented by the following formula (A-1) and/or an isoindoline compound represented by the following formula (A-3).

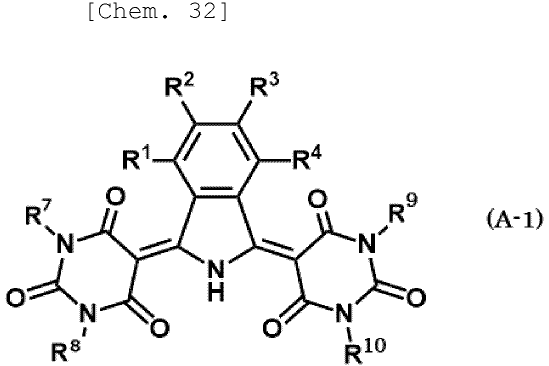

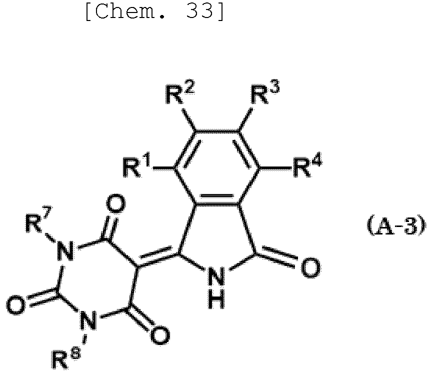

In the formula (A-1) and the formula (A-3), R¹ to R⁴ represent the same as R¹ to R⁴ above. R⁷ to R¹⁰ represent the same as R⁷ to R¹⁰ above.

The percentage of the isoindoline compound represented by the formula (A-3) in the composition is preferably from 0 to 30% by mass, more preferably from 0.001 to 15% by mass, more preferably from 0.01 to 10% by mass, more preferably from 0.1 to 7% by mass, and even more preferably from 0.5 to 5.0% by mass.

As described above in the <Method For Producing Isoindoline Compound Represented By (I-2)>, bio-derived furan and bio-derived maleic anhydride are used as a raw material, and the compound represented by the formula (DA) is obtained by the Diels-Alder (DA) reaction, and the compound represented by the formula (DA) is dehydroaromatized and aminated with urea to produce an isoindoline compound containing a radiocarbon atom ¹⁴C and represented by the formula (I-2). In that case, in the composition containing the isoindoline compound represented by the formula (I-2), there may be present an isoindoline compound represented by the following formula (I-1), which has been produced in the production process.

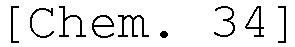

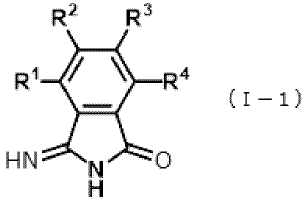

In the formula (I-1), R¹ to R⁴ represent the same as R¹ to R⁴ above.

When the composition in which an isoindoline compound represented by the formula (I-1) above is also present in addition to the isoindoline compound represented by the formula (I-2) is reacted with barbituric acid represented by the formula (B) described in <Reaction G> described above of (Method For Producing Isoindoline Compound Containing Group Represented By Formula (A)) described above, the isoindoline compound represented by the formula (A-3) is also produced in addition to the isoindoline compound represented by the formula (A-1).

In addition to the isoindoline compound represented by the formula (A-1), the present invention can provide a composition also containing the isoindoline compound represented by the formula (A-3). The pigment composition has an excellent color intensity and exhibits a pigment performance equal to or better than that of commercially available non-bioisoindoline pigment compositions. Accordingly, a Y139 bio-isoindoline-based yellow pigment composition having an excellent color intensity can be provided.

More preferred embodiments of the composition of the present invention include a composition containing an isoindoline compound represented by the following formula (A-1-1) and an isoindoline compound represented by the following formula (A-3-1).

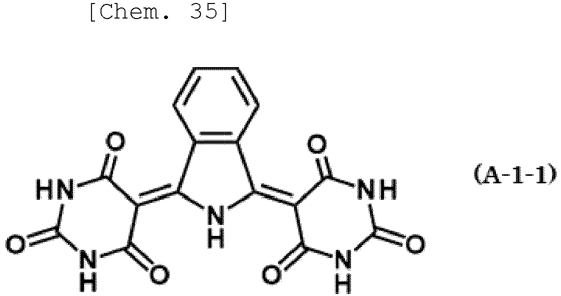

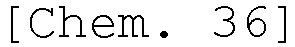

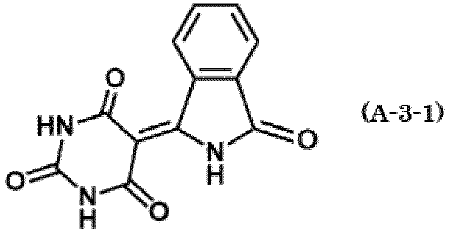

The percentage of the isoindoline compound represented by the formula (A-3-1) in the composition is preferably from 0 to 30% by mass, more preferably from 0.001 to 15% by mass, more preferably from 0.01 to 10% by mass, more preferably from 0.1 to 7% by mass, and more preferably from 0.5 to 5.0% by mass.

The BET value for the composition of the present invention is preferably from 20 to 100 m²/g, preferably from 30 to 95 m²/g, preferably from 40 to 90 m²/g, and preferably from 50 to 85 m²/g. In particular, the BET value is preferably from 20 to 80 for ink applications, preferably from 40 to 90 for paint applications, preferably from 50 to 90 for resin applications, and preferably from 50 to 100 for color filter applications. Note that the BET value can be measured by weighing 0.1 g of pigment in a measuring cell and setting it on a specific surface area meter (Macsorb 1208).

### (Application Of Isoindoline Compound Of Present Invention)

The isoindoline compound of the present invention obtained by the above method, having a group represented by the formula (A), and containing a radiocarbon atom ¹⁴C, and the composition containing the isoindoline compound constitute an isoindoline-based yellow pigment such as Y139 and Y185.

The isoindoline compound of the present invention contains biomass-derived carbon, which contributes to reducing an environmental load through carbon neutrality.

In addition, the isoindoline compound of the present invention is obtained by the above production method, and therefore the number and positions of the various types of functional groups can be controlled by changing the type of furan or changing to other types of dienes.

The isoindoline compound of the present invention and the isoindoline-based yellow pigment including the composition having the isoindoline compound can be used as colored compositions, molding compositions, and the like.

In addition, the isoindoline compound of the present invention and the isoindoline-based yellow pigment including the composition having the isoindoline compound are mixed with other resins, rubbers, additives, solvents, pigments and dyes, or the like, as needed, and are adjusted for use as coating materials or printing markers for cosmetics, pharmaceuticals or agricultural chemicals, stationery, writing materials, printing inks, inkjet inks, metallic inks, paints, plastic colorants, toners (color toners), color filters, and the like.

### <Colored Composition>

The colored composition preferably includes the isoindoline compound of the present invention and the isoindoline-based yellow pigment composition including the composition having the isoindoline compound, and a dispersion medium.

### <<Dispersion Medium>>

Examples of the dispersion media includes resins and solvents. Examples of the resins include resin-type dispersants and binder resins. Examples of the solvents include water and organic solvents. Note that low molecular weight dispersants such as surfactants can be used, as needed.

Examples of the type of resins in the resin-type dispersant include styrene-(meth)acrylic acid copolymers, (meth)acrylic acid-(meth)acrylic acid alkyl ester copolymers, styrene-(meth)acrylic acid-(meth)acrylic acid alkyl ester copolymers, styrene-α-methylstyrene-(meth)acrylic acid copolymers, styrene-α-methylstyrene-(meth)acrylic acid-(meth)acrylic acid alkyl ester copolymers, poly(meth)acrylic acid, vinylnaphthalene-(meth)acrylic acid copolymers, styrene-maleic acid copolymers, maleic acid-maleic anhydride copolymers, **α-**olefin-maleic (anhydride) copolymers, α-olefin-maleic (anhydride)-polyalkylene glycol allyl ether copolymers, vinylnaphthalene-maleic acid copolymers, polyester modified (meth)acrylic acid polymers, and salts thereof.

In addition, examples of the form of the resin in the resin-type dispersant includes water-soluble resins and emulsions (water-insoluble resins).

A binder resin may be, for example, polyolefin resins, polyester resins, styrene copolymers, acrylic resins, and modified resins thereof. Specific examples thereof include polyolefin resins such as polyethylene such as high-density polyethylene (HDPE), linear low-density polyethylene (L-LDPE), and low-density polyethylene (LDPE), and polypropylene; polyester resins such as polyethylene terephthalate; styrene copolymers such as styrene-p-chlorostyrene copolymers, styrene-vinyl toluene copolymers, styrene-vinylnaphthalene copolymers, styrene-acrylic acid ester copolymers, styrene-methacrylic acid ester copolymers, styrene-α-methyl chloromethacrylate copolymers, styrene-acrylonitrile copolymers, styrene-vinyl methyl ether copolymers, styrene-vinyl ethyl ether copolymers, styrene-vinyl methyl ketone copolymers, styrene-butadiene copolymers, styrene-isoprene copolymers, and styrene-acrylonitrile-indene copolymers; acrylic resins such as acrylic resins and methacrylic resins; polyvinyl chloride, phenol resins, natural modified phenol resins, natural resin modified maleate resins, polyvinyl acetate, silicone resins, polyurethane resins, ethylene-vinyl acetate copolymer resins, vinyl acetate resins, nitrocellulose resins, polyamide resins, epoxy resins, xylene resins, polyvinyl butyral resins, polyvinyl acetal resins, cellulose ester resins, alkyd resins, rosin-based resins, ketone resins, cyclized rubber, chlorinated polyolefin resins, terpene resins, coumarone indene resins, alkyd resins, amino resins, petroleum resins, and modified resins thereof.

Organic solvents can be classified into water-soluble solvents and water-insoluble solvents.

Examples of the water-soluble solvents include ethanol, n-propanol, isopropanol, isobutanol, ethylene glycol, diethylene glycol, triethylene glycol, propylene glycol, and glycerin. Examples of the water-insoluble solvents include toluene, xylene, butyl acetate, methyl acetate, methyl ethyl ketone, methyl isobutyl ketone, butyl alcohol, and aliphatic hydrocarbon.

Each of the materials constituting the colored composition can be used alone or in combination of two or more kinds.

### <Molding Composition>

A molding composition contains the colored composition (pigment composition and resin). The molding composition preferably contains a thermoplastic resin in the resin. The molding composition containing a thermoplastic resin is preferably melted, mixed, and molded into a desired shape to produce a molded body.

Examples of the thermoplastic resins include a homopolymer or a copolymer using ethylene, propylene, butylene, styrene, or the like as a monomer component. More specifically, examples thereof include polyolefin resins such as polyethylene such as high-density polyethylene (HDPE), linear low-density polyethylene (L-LDPE), and low-density polyethylene (LDPE), polypropylene, and polybutylene. Other specific examples of useful resins include polyester resins such as polyethylene terephthalate, polyamide resins such as nylon 6 and nylon 66, polystyrene resins, and thermoplastic ionomer resins. Among these, polyolefin resins and polyester resins are preferred. Note that the number average molecular weight of the thermoplastic resin is preferably greater than 30,000 and 200,000 or less.

The molding composition can contain wax. Wax includes low molecular weight polyolefins. These are a polymer of an olefin monomer such as ethylene, propylene, and butylene, and may be a block or random copolymer, or a terpolymer. Specifically, wax is polymers of α-olefins such as low-density polyethylene (LDPE), high-density polyethylene (HDPE), and polypropylene (PP).

The number average molecular weight of the wax is preferably from 1,000 to 30,000, and more preferably from 2,000 to 25,000. Within this range, wax migrates moderately to a surface of the molded body, resulting in an excellent balance between sliding properties and bleed-out control.

The melting point of the wax is preferably from 60 to 150°C, and more preferably 70 to 140°C. Within this range, excellent processability is achieved when melting and kneading the thermoplastic resin and wax.

The molding composition can contain other additives. Other additives are materials commonly used in the technical field of the molded body, and examples thereof include antioxidants, light stabilizers, dispersants, metallic soaps, antistatic agents, flame retardants, lubricants, fillers, and colorants other than the isoindoline compounds of the present invention.

The molding composition can be produced, for example, as a masterbatch containing the isoindoline compound in a high concentration.

The masterbatch, for example, is preferably made by melting and kneading the thermoplastic resin and the pigment composition, and then molding them into an optional shape for easy use in a next process. The masterbatch and a diluted resin (for example, the thermoplastic resin used in the masterbatch) can then be melted and kneaded to form a molded body having a desired shape. Examples of the shape of the masterbatch include a pellet shape, a powder shape, and a plate shape. Note that, in order to prevent an agglomeration of the pigment composition, it is preferable to produce a dispersion obtained by melting and kneading the pigment composition and wax together in advance, and then produce the masterbatch by melting and kneading the dispersion together with the thermoplastic resin. As the devices used for dispersions, for example, a blend mixer, a three-roll mill, and the like are preferably used.

Examples of applications of the molding composition include plastic molded bodies, sheets, and films.

### <Toner>

A toner contains a colored composition (pigment composition and resin). The resin in the toner is called a binding resin, and is preferably a thermoplastic resin. The toner includes a dry toner and a wet toner. For example, the dry toner is made by melting and kneading the pigment composition and the binding resin, followed by cooling, and grinding and classification processes. Next, the production can be carried out by a post-processing process in which additives are blended and mixed.

Examples of the binding resins include styrene-p-chlorostyrene copolymers, styrene-vinyltoluene copolymers, styrene-vinylnaphthalene copolymers, styrene-(meth)acrylic ester copolymers, styrene-α-methyl chloromethacrylate copolymers, styrene-acrylonitrile copolymer, styrene-vinyl methyl ether copolymers, styrene-vinyl ethyl ether copolymers, styrene-vinyl methyl ketone copolymers, styrene-butadiene copolymers, styrene-isoprene copolymers, styrene-acrylonitrile-indene copolymers, polyvinyl chloride, phenolic resins, natural modified phenol resins, natural resin-modified maleate resins, (meth)acrylic resins, polyvinyl acetate, silicone resins, polyester resins, polyurethane, polyamide resins, furan resins, epoxy resins, xylene resins, polyvinyl butyral, terpene resins, coumarone indene resins, and petroleum-based resins.

Among these, polyester resins and styrene-based copolymers are preferred, and polyester resins are more preferred. The pigment composition has particularly excellent compatibility with polyester resins, so that the isoindoline compound is uniformly and finely dispersed in the toner, resulting in high-quality toners.

The weight average molecular weight (Mw) of the polyester resin is preferably 5,000 or more, more preferably from 10,000 to 1,000,000, and even more preferably from 20,000 to 100,000. When the polyester resin having a moderate Mw is used, the toner having excellent offset resistance and low-temperature fixing properties can be obtained.

The acid value of the polyester resin is preferably from 10 to 60 mgKOH/g, and more preferably from 15 to 55 mgKOH/g. When the polyester resin having a moderate acid value is used, the mold releasing agent is easily prevented from being released, and a decrease in an image density in a high humidity environment is unlikely to occur.

The toner can further contain a charge control agent. The use of the charge control agent makes it easier to obtain a toner having a stable charge amount. The charge control agent can be selected as appropriate from positive and negative charge control agents to be used.

The toner can contain the mold releasing agent. Examples of the mold releasing agents include hydrocarbon-based waxes such as polypropylene wax, polyethylene wax, and Fischer-Tropsch wax, synthetic ester waxes, and natural ester-based waxes such as carnaba wax and rice wax.

The lubricants, fluidizing agents, polishing agents, conductivity-imparting agents, image peeling inhibitors, or the like may be added to the toner, as needed.

The toner can also be used as a one-component type developer or a two-component type developer. The two-component type developer can further contain a carrier.

Examples of the carriers include magnetic powders such as iron powder, ferrite powder, and nickel powder, and coating treatment objects having their surfaces coated with resins or the like. Examples of the resins that coat the carrier surface include styrene-(meth)acrylic ester copolymers, (meth)acrylic ester copolymers, fluorine-containing resins, silicone-containing resins, polyamide resins, ionomer resins, and polyphenylene sulfide resins.

### <Paint>

A paint contains the colored composition (pigment composition, resin, and solvent).

Examples of the resins include the thermosetting resins and the thermoplastic resins. The thermosetting resin is preferably a resin having a glass transition temperature of 10°C or higher. Examples of the types of the thermosetting resins include acrylic resins, polyester, and polyurethane. In addition, the thermosetting resin preferably has a functional group that can react with a curing agent. Examples of the functional groups include a carboxyl group and a hydroxyl group. Examples of the curing agents include isocyanate curing agents, epoxy curing agents, aziridine curing agents, and amine curing agents.

The thermoplastic resin is preferably a resin having a glass transition temperature of 30°C or higher. Examples of the thermoplastic resins include nitrocellulose and polyester. Note that the thermosetting resin and the thermoplastic resin can be used together.

Among solvents, examples of the water-insoluble solvent include toluene, xylene, butyl acetate, methyl acetate, methyl ethyl ketone, methyl isobutyl ketone, butyl alcohol, and aliphatic hydrocarbon.

Among solvents, examples of the water-soluble solvents include water, monovalent alcohols, divalent alcohols, and glycols. Examples of the water-soluble solvents include ethanol, n-propanol, isopropanol, isobutanol, ethylene glycol, diethylene glycol, triethylene glycol, propylene glycol, and glycerin. The water-soluble solvents also include water-dilutable monoethers derived from polyhydric alcohols. Specific examples thereof include methoxypropanol or methoxybutanol. Also, examples of the water-soluble solvents include water-dilutable glycol ethers such as butyl glycol or butyl diglycol. Note that in a case where the solvent contains water, the paint is called a water-based paint.

The paint can further contain known additives.

The applications of the paint, for example, include coatings for metal and coatings for plastic.

### <Printing Ink>

The printing ink contains the colored composition (pigment composition, resin, and solvent). The printing ink is an ink other than inkjet inks, and examples thereof include offset printing inks, flexographic printing inks, gravure printing inks, silk-screen printing inks, and color filter inks. Note that in a case where the solvent contains water, the printing ink is called a water-based printing ink.

Examples of the resins include rosin resins, rosin-modified phenolic resins, polyurethane, nitrocellulose, acrylic resins, styrene-acrylic resins, and petroleum resins.

Among solvents, examples of the water-insoluble solvent include toluene, xylene, butyl acetate, methyl acetate, methyl ethyl ketone, methyl isobutyl ketone, butyl alcohol, and aliphatic hydrocarbon.

Among solvents, examples of the water-soluble solvents include ethanol, n-propanol, isopropanol, isobutanol, ethylene glycol, diethylene glycol, triethylene glycol, propylene glycol, and glycerin. The water-soluble solvents also include water-dilutable monoethers derived from polyhydric alcohols. Examples thereof include methoxypropanol or methoxybutanol. Also, examples of the water-soluble solvents include water-dilutable glycol ethers such as butyl glycol or butyl diglycol.

The printing ink can further contain glitter materials. The glitter material has particles having an average thickness from 0.5 to 10 µm and an average particle size from 5 to 50 µm, and examples thereof include metal flakes, mica, and coated glass flakes. Examples of the metal flakes include aluminum flakes and gold powder. Examples of mica include ordinary mica and coated mica. Examples of the coated glass flakes include glass flakes coated with metal oxides such as titanium oxide.

The printing ink can further contain known additives.

### <Inkjet Ink>

The inkjet ink contains the pigment composition and the resin, and preferably further contains the solvent. The inkjet ink can be broadly classified into a (solvent-based) inkjet ink, a water-based inkjet ink, and a solventless inkjet ink, depending on the presence or absence of the solvent and its type.

Hereinafter, The description will focus on a water-based inkjet ink.

The resin used in the water-based inkjet ink is important to obtain the fixation of ink on a printed material (substrate).

Examples of the type of the resin include acrylic-based resins, styrene-acrylic-based resins, polyester-based resins, polyamide-based resins, and polyurethane-based resins. Examples of the form of the resin include water-soluble resins and emulsion particles. Among these, emulsion particles are preferred. The emulsion particles include single-composition particles, core-shell type particles, and the like, which can optionally be selected and used. When the emulsion particles are used, it is easy to lower the viscosity of the water-based inkjet ink, and a recorded material having excellent water resistance can be easily obtained. The resin can be used with an acidic functional group neutralized by pH adjusters such as ammonia, various amines, and various inorganic alkalis, as needed.

Examples of the solvents include water-insoluble solvents, water, and water-soluble solvents. The water-soluble solvent includes glycol ethers and diols, and these solvents penetrate into substrates very quickly, and they penetrate quickly even into low-liquid absorbing and non-liquid absorbing substrates such as coated paper, art paper, vinyl chloride sheets, films, and fabrics. As a result, drying during printing is quick and accurate printing can be achieved. It also acts as a wetting agent due to its high boiling point.

The water-soluble solvent is important for preventing drying and solidification of the water-based inkjet ink at the nozzle portion of the printer head and for achieving ink discharge stability. Examples of the water-soluble solvents include ethylene glycol, diethylene glycol, propylene glycol, triethylene glycol, polyethylene glycol, glycerin, tetraethylene glycol, dipropylene glycol, ketone alcohol, diethylene glycol monobutyl ether, ethylene glycol monobutyl ether, ethylene glycol monoethyl ether, 1,2-hexanediol, N-methyl-2-pyrrolidone, substituted pyrrolidone, 2,4,6-hexanetriol, tetrafurfuryl alcohol, and 4-methoxy-4methylpentanone.

The inkjet ink can further contain additives. Examples of the additives include drying accelerators, penetrants, preservatives, chelating agents, and pH adjusters.

The inkjet ink is prepared by blending and mixing each material. Mixing can be carried out by a stirrer using blades, various dispersing machines, emulsifying machines, and the like. The order of addition of each material and the mixing method are optional.

After mixing, the inkjet ink is preferably filtered or centrifuged to remove coarse particles. This improves ejecting properties from the inkjet printer. Known methods of filtration and centrifugation can be used.

Various inkjet systems can be used for the inkjet ink. Examples of the inkjet systems include a charge-controlled type, a continuous jetting type such as a spray type, a piezo system, a thermal system, and an electrostatic suction system.

### [Example]

Hereinafter, the present invention will be described in more detail with reference to Examples, but the present invention is not limited to these Examples. In addition, "%" in the compositions in the following Examples means '% by mass'.

### (Synthesis Example 1) Synthesis of DA Intermediate

Under a nitrogen atmosphere, 12 g of biomass-derived maleic anhydride was dissolved in diethyl ether (250 mL) in a reactor equipped with a stirrer device, 10 g of biomass-derived furan was added thereto, and the reaction was carried out at room temperature and 0.25 MPa for 24 hours. The white crude was then filtered out with a filter and dried in vacuum to obtain 20 g of a DA intermediate.

### (Synthesis Example 1) Bio-DII

14 g of acetic anhydride was added to 74 g of methanesulfonic acid, and 5 g of the DA intermediate was slowly added thereto while cooling in an ice bath. The reaction mixture was stirred at room temperature for 2 hours and then heated at 80°C for 4 hours. The reaction mixture was extracted with toluene (3 × 50 mL). The added toluene was washed with salt water and sodium bicarbonate and evaporated under reduced pressure. The resulting needle white substance was mixed with 13 g of urea, 6 g of ammonium nitrate, and 0.03 g of molybdenum in 10 mL of alkylbenzene. After heating the reaction mixture at 165°C for 5 hours, alkylbenzene was removed by vacuum distillation. 30 mL of methanol was added to the reaction mixture and distilled for 15 minutes, and the reaction mixture was then cooled to room temperature and filtered. The resulting solid was dispersed in 10 mL of water, and 3 g of ammonia water and 4.4 mL of 25% caustic soda were added thereto. The solution was cooled in an ice bath, and the precipitated solid was filtered and washed with water. The solid obtained was dried under reduced pressure to obtain 2.9 g of a compound containing a radiocarbon C¹⁴ in the molecule.

### (Example 1) Synthesis of Isoindoline Compound

To a reaction vessel equipped with a cooling tube, 1.2 g of Bio-DII obtained in Synthesis Example 1, 2.7 g of barbituric acid, 19 mL of water, and 1 mL of acetic acid at 5% by mass concentration were added and heated to 90°C while stirring. After stirring for 1.5 hours, the mixture was cooled to room temperature, the crude material was filtered out with a filter and washed with water, IPA, and water in that order, and the resulting solid was then dried in an oven to obtain 2.7 g of an isoindoline compound containing a radiocarbon C¹⁴ in the molecule.

### (Comparison Example 1)

Commercially available Y139 (Fanchon Yellow 279-5139) (manufactured by Sun Chemical) was purchased and used.

### (Mass Spectrometry)

The pigment compositions containing the isoindoline compounds obtained in Example 1 and Comparative Example 1 described above were analyzed by mass spectrometry. For the analysis method, 5 mg of the obtained pigment composition was dissolved in THF and subjected to FD-MS JMS-T100GC (manufactured by JEOL Ltd.). The results are shown in the table below.

**[Table 1]**

| *m*/*z* | 183 | 238 | 257 | 280 | 312 | 323 | 334 | 367 | 384 | 568 | 640 | 896 |
|---|---|---|---|---|---|---|---|---|---|---|---|---|
| Example 1 | 0.2 | 0.1 | 3 | - | 0.1 | - | 0.1 | 96.6 | - | - | - | - |
| Comparative Example 1 | - | - | 0.2 | 0.1 | - | 0.2 | - | 98.8 | 0.1 | 0.1 | 0.3 | 0.2 |

The results in Table 1 above showed that the pigment composition of Example 1 contained 96.6% of the compound represented by the formula (A-1-1) and 3.0% of the compound represented by the formula (A-3-1).

### (TEM Observation)

For the pigment compositions containing the isoindoline compounds obtained in Example 1 and Comparative Example 1 described above, a TEM observation was carried out to check the state of the particles.

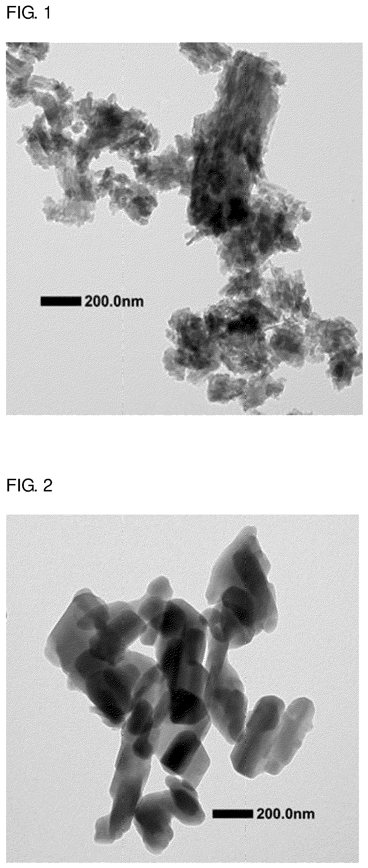

The results of the TEM observation of Example 1 are shown in FIG. 1, and the results of the TEM observation of Comparative Example 1 are shown in FIG. 2.

The specific surface area (BET value) of Example 1 was 74 m²/g, while the BET value of Comparative Example 1 was 18 m²/g.

### (Accelerator Mass Spectrometry (AMS) Method)

A ¹⁴C-AMS dedicated device (manufactured by NEC Corporation) based on a tandem accelerator was used to measure ¹⁴C counts, a ¹³C concentration (¹³C/¹²C), and a ¹⁴C concentration (¹⁴C/¹²C).

In the measurement, oxalic acid (HOxII) provided by the National Institute of Standards and Technology (NIST) was used as a standard sample. The measurements of this standard sample and a background sample were also carried out at the same time. The percent Modern Carbon (pMC) is a ratio of the ¹⁴C concentration of the sample carbon to the standard modern carbon.

The analytical results of the AMS method for Example 1 and Comparative Example 1 are shown in Table 2 below.

**[Table 2]**

| Sample name | pMC (%) |
|---|---|
| Example 1 | 51.63 ± 0.21 |
| Comparative Example 1 | 0.20 ± 0.02 |

### (Planographic ink test)

Using the respective pigment compositions of Example 1 and Comparative Example 1, 1 part of the pigment composition and 4 parts of MG63 varnish were kneaded using a Hoover muller, 10 times FG79 white was added, the resulting ink was spread on paper and the L*a*b*C*h* value and the color intensity were measured using a spectrophotometer (Datacolor 650). As for the obtained L*a*b*C*h* values, the values of the difference between Example 1 and Comparative Example 1 (Example 1 - Comparative Example 1) are shown in Table 3 below.

**[Table 3]**

| | L* | a* | b* | C* | h* |
|---|---|---|---|---|---|
| Difference between Example 1 and Comparative Example 1 | 0.53 | -5.97 | 12.18 | 10.79 | 11.61 |

When the color intensity of the pigment composition of Example 1 was taken as 100, the color intensity of the pigment composition of Comparative Example 1 was 66, and as to the color intensity of the offset ink, the color intensity of the Y139 pigment composition of the present invention was 34% higher compared to that of the commercially available Y139 pigment composition.

## Claims

1. An isoindoline compound comprising a radiocarbon atom ¹⁴C and a group represented by the following formula (A): 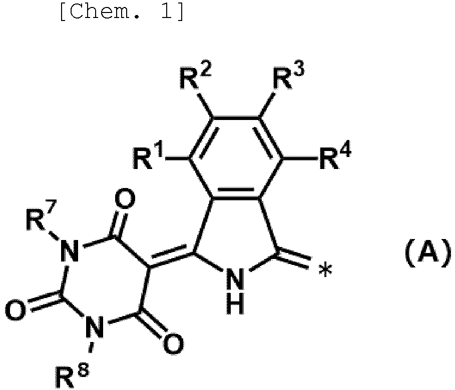 in the formula (A), R¹ to R⁴ each independently represent a hydrogen atom, a linear or branched alkyl group having 1 to 12 carbon atoms, a fluorine atom, a chlorine atom, a bromine atom, an iodine atom, or a phenyl group that is optionally substituted with an alkyl group, R² and R³ are optionally ring-closed to form a 5- to 8-membered ring, R⁷ and R⁸ each independently represent a hydrogen atom, a linear or branched alkyl group having 1 to 12 carbon atoms, or a phenyl group that is optionally substituted with an alkyl group, and * represents a bonding hand.

2. The isoindoline compound according to claim 1, wherein the isoindoline compound comprising a group represented by the formula (A) is an isoindoline compound represented by the following formula (A-1) or the following formula (A-2): 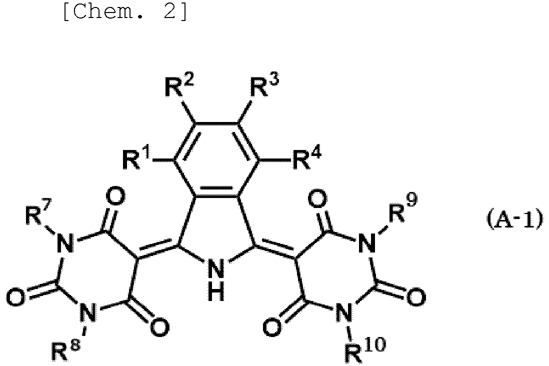 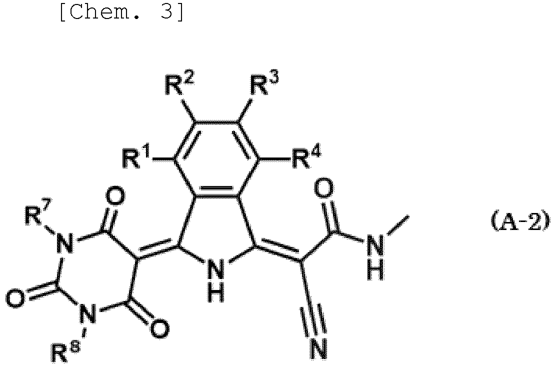 in the formula (A-1) and the formula (A-2), R¹ to R⁴ represent same as R¹ to R⁴ above, R⁷ and R⁸ represent same as R⁷ and R⁸ above, and R⁹ and R¹⁰, similar to R⁷ and R⁸ above, each independently represent a hydrogen atom, a linear or branched alkyl group having 1 to 12 carbon atoms, or a phenyl group that is optionally substituted with an alkyl group.

3. The isoindoline compound according to claim 2, wherein the isoindoline compound represented by the formula (A-1) is an isoindoline compound represented by the following formula (A-1-1), or the isoindoline compound represented by the formula (A-2) is an isoindoline compound represented by the following formula (A-2-1):

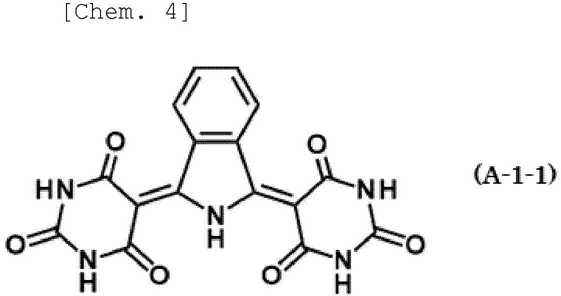

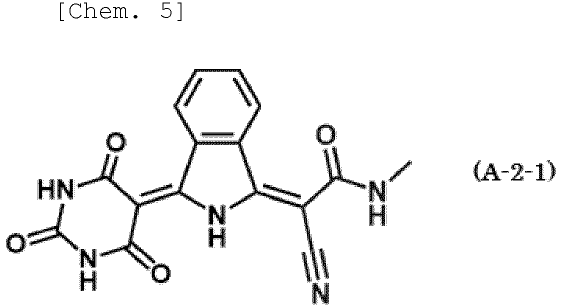

4. A composition comprising the isoindoline compound comprising a group represented by the formula (A) according to claim 1.

5. The composition according to claim 4, used as a composition for pigments.

6. The composition according to claim 4, comprising at least two kinds of the isoindoline compounds comprising a group represented by the formula (A).

7. The composition according to claim 4, comprising an isoindoline compound represented by the following formula (A-1) and an isoindoline compound represented by the following formula (A-3): 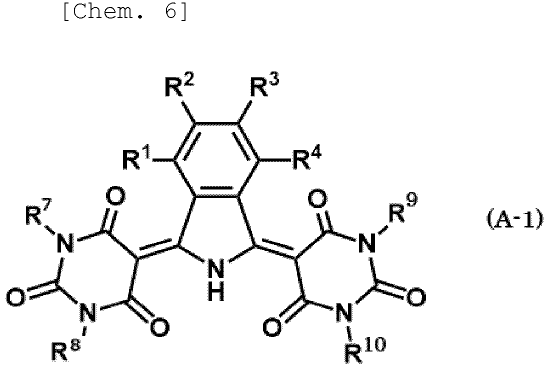 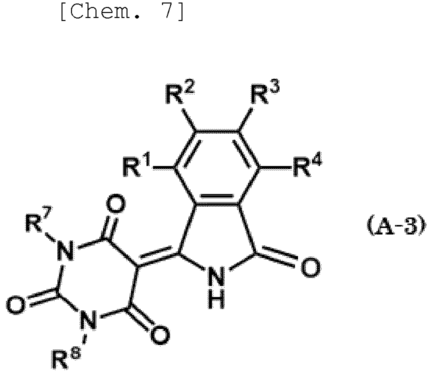 in the formula (A-1) and the formula (A-3), R¹ to R⁴ each independently represent a hydrogen atom, a linear or branched alkyl group having 1 to 12 carbon atoms, a fluorine atom, a chlorine atom, a bromine atom, an iodine atom, or a phenyl group that is optionally substituted with an alkyl group, R² and R³ are optionally ring-closed to form a 5- to 8-membered ring, and R⁷ to R¹⁰ each independently represent a hydrogen atom, a linear or branched alkyl group having 1 to 12 carbon atoms, or a phenyl group that is optionally substituted with an alkyl group.

8. The composition according to claim 7, wherein a ratio of the isoindoline compound represented by the formula (A-3) in the composition is 0.5 to 10.0% by mass.

9. The composition according to claim 7, wherein the isoindoline compound represented by the formula (A-1) is an isoindoline compound represented by the following formula (A-1-1), and the isoindoline compound represented by the formula (A-3) is an isoindoline compound represented by the following formula (A-3-1):

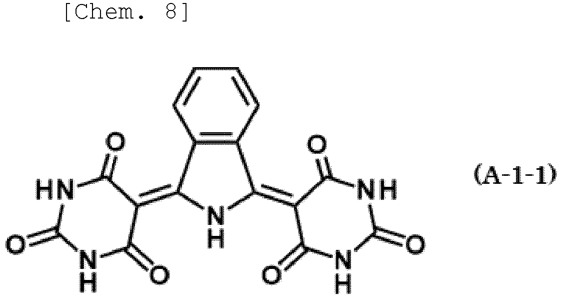

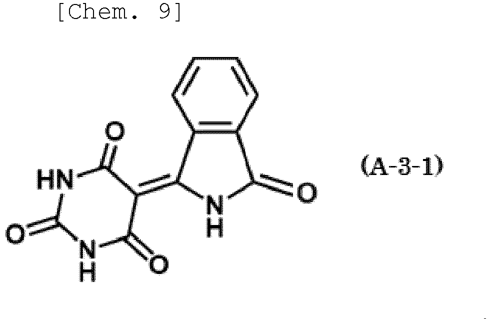

10. A method for producing an isoindoline compound comprising a radiocarbon atom ¹⁴C and a group represented by the following formula (A): 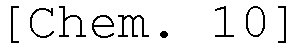 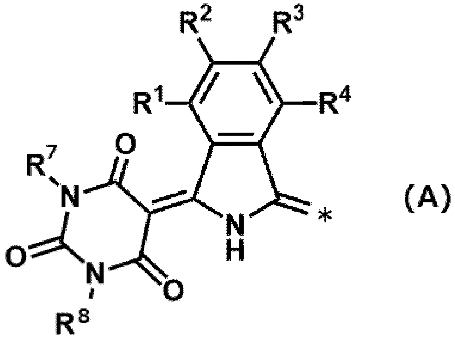
in the formula (A), R¹ to R⁴ each independently represent a hydrogen atom, a linear or branched alkyl group having 1 to 12 carbon atoms, a fluorine atom, a chlorine atom, a bromine atom, an iodine atom, or a phenyl group that is optionally substituted with an alkyl group, R² and R³ are optionally ring-closed to form a 5- to 8-membered ring, R⁷ and R⁸ each independently represent a hydrogen atom, a linear or branched alkyl group having 1 to 12 carbon atoms, or a phenyl group that is optionally substituted with an alkyl group, and * represents a bonding hand,
the method comprising reacting a compound represented by the following formula (I-2) with barbituric acid represented by the following formula (B) under an acid-free solvent or under a solvent containing at least one kind of acid selected from the group consisting of hydrochloric acid, sulfuric acid, methanesulfonic acid, p-toluenesulfonic acid, acetic acid, triflic acid, phosphoric acid, polyphosphoric acid, pyrophosphoric acid, and nitric acid to obtain an isoindoline compound comprising a group represented by the formula (A): 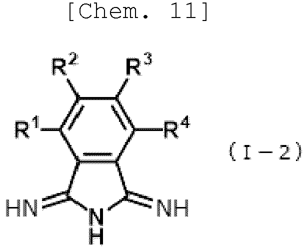
in the formula (I), R¹ to R⁴ represent same as R¹ to R⁴ above, and 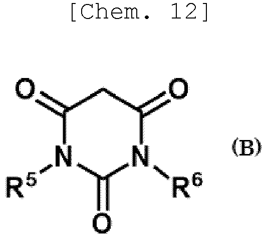
in the formula (B), R⁵ and R⁶ each independently represent a hydrogen atom, a linear or branched alkyl group having 1 to 12 carbon atoms, or a phenyl group that is optionally substituted with an alkyl group.

11. The method for producing an isoindoline compound according to claim 10, wherein
the compound represented by the formula (I-2) is obtained by obtaining a compound represented by the following formula (PA) from a compound represented by the following formula (DA), 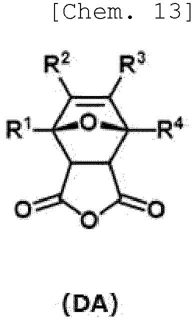
in the formula (DA), R¹ to R⁴ represent same as R¹ to R⁴ above, 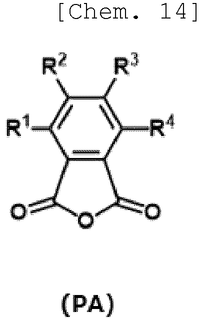
in the formula (PA), R¹ to R⁴ represent same as R¹ to R⁴ above, and
obtaining an isoindoline compound represented by the formula (I-2) from the compound represented by the formula (PA).

12. The method for producing an isoindoline compound according to claim 10, wherein the solvent is any one selected from the group consisting of H₂O, dimethylacetamide (DMA), dimethylformamide (DMF), dimethyl sulfoxide (DMSO), MeOH, EtOH, isopropyl alcohol (IPA), and tBuOH.
